# EUROPÄISCHE PATENTSCHRIFT

(11) **EP 0 602 096 B1**
(45) Veröffentlichungstag und Bekanntmachung des Hinweises auf die Patenterteilung: **27.12.1995**
(21) Anmeldenummer: 92918309.3
(22) Anmeldetag: 03.09.1992
(51) Int. Cl.: B09B 3/00

(54) **VERFAHREN ZUR MIKROBIOLOGISCHEN BODENREINIGUNG**
MICROBIOLOGICAL SOIL DECONTAMINATION PROCESS
PROCEDE DE NETTOYAGE MICROBIOLOGIQUE D'UN SOL

(30) Priorität: 04.09.1991 DE 4129363
(43) Veröffentlichungstag der Anmeldung: 22.06.1994
(73) Patentinhaber: Bergwerksverband GmbH, D-45307 Essen (DE)
(72) Erfinder: BEYER, Michael, D-4300 Essen 17 (DE); NITSCHKE, Volker, D-4300 Essen 11 (DE); VAN AFFERDEN, Manfred, D-4630 Bochum 1 (DE); SCHULZ, Peter, D-4300 Essen 14 (DE)
(86) Internationale Anmeldenummer: EP9202030
(87) Internationale Veröffentlichungsnummer: WO9304794

(56) Entgegenhaltungen:
- EP-A- 0 290 661
- EP-A- 0 379 121
- EP-A- 0 432 651
- WO-A-91/02565
- DE-A- 3 818 398
- DE-A- 3 921 066
- DE-C- 3 920 827
- DE-C- 4 001 558
- NL-A- 8 802 728
- SYLLABUS SYMPOSIUM BIOTECHNOLOGISCHE BODEMSANERING, 12 Juni 1990, Jaarbeurs congrescentrum, Utrecht, NL KIVI/NIRIA, Den Haag, NL KLEIJNTJENS ET AL: 'De ontwikkeling van een slurry proces voor de biologische reiniging van vervuilde grond'

## Beschreibung

Die Erfindung betrifft ein Verfahren zur mikrobiologischen Bodenreinigung, wobei der verunreinigte Boden ausgehoben und in eine Grobfraktion und eine Feinfraktion getrennt wird, die Feinfraktion in einem Bioreaktor dekontaminiert wird, Betriebswasser in den Kreislauf zurückgeführt wird und der dekontaminierte Boden, gegebenenfalls nach Wiederzumischung der Grobfraktion, wieder angefüllt wird.

Ein solches verfahren ist aus "Entwurf eines Slurry-Prozesses zur biotechnologischen Altlastensanierung", Altlastensanierung '90, Dritter Internationaler KfK/TNO-Kongress über Altlastensanierung, 10. - 14. Dezember 1990, Karlsruhe, Bundesrepublik Deutschland, Band II, Kluwer Academic Publishers, Dordrecht/Boston/London, Seiten 1103 und 1104, bekannt geworden. Bei diesem Slurry-Prozess ist es nachteilig, daß vier Bioreaktoren benötigt werden und zwar ein flüssigkeitsbewegter Reaktor und drei gasbewegte Reaktoren, um durch Öl verschmutzte Böden zu reinigen. Zur Beseitigung von polyzyklischen aromatischen Kohlenwasserstoffen aus Böden mit hohem Schluffanteil ist dieses Verfahren nicht vorgesehen und auch nicht geeignet.

Weiterhin sind aus der DE 37 33 341 A1 ein Verfahren und eine Einrichtung zur mikrobiologischen Bodenreinigung bekannt, um mit Kohlenwasserstoffen, insbesondere mit Mineralölen kontaminierte Böden, durch mikrobielle Oxidation zu reinigen, wobei den Böden neben Sauerstoff, Wasser, lösliche Nährstoffe sowie lösliche oder dispergierte Tenside, z.B. in einem "hydrodynamischen" Bioreaktor, zugeführt werden, um eine vollständige Mineralisierung der Mineralöle durch Veratmung zu CO₂ und H₂0 zu bewirken, wobei eine Regelung von Temperatur, pH-Wert, Sauerstoffkonzentration und Begasungsrate vorgesehen ist. Nachteilig ist hierbei, daß die vorgesehene durch Belüftung eingetragene Sauerstoffmenge bei weitem nicht dem Sauerstoffbedarf zur biologischen Mineraloxidaton entspricht. Darüberhinaus ist es nachteilig, daß das Verfahren wegen der Zugabe der Tenside verteuert wird und eine vergleichsweise aufwendige Regeleinrichtung benötigt wird. Zur Beseitigung von polyzyklischen aromatischen Kohlenwasserstoffen aus Böden mit hohem Schluffanteil ist dieses Verfahren nicht vorgesehen und auch nicht geeignet.

Außerdem ist dem Fachmann bekannt, daß Rührkesselreaktoren mit maximal 10 % und pneumatisch betriebene Reaktoren (Blasensäulen / Airliftreaktoren) mit maximal 25 % Feststoffgehalt wirtschaftlich betrieben werden können.

Schließlich ist aus der DE 39 20 827 C1 eine Reinigungsanlage für kontaminierte Böden in Form einer gattungsfremden Regenerationsmiete zur Behandlung von naturfeuchten Böden bekannt, die einen Bewässerungskreislauf und einen Belüftungskreislauf aufweist und über Regeleinrichtungen verfügt, die die Feuchtigkeit und die mikrobielle Aktivität konstant halten, wobei wechselweise aerobe und anaerobe Behandlungsbedingungen Anwendung finden können. Bei dieser Anlage ist es schwierig, die Wetterbedingungen zu neutralisieren. Zur Beseitigung von polyzyklischen aromatischen Kohlenwasserstoffen aus Böden mit hohem Schluffanteil ist dieses Verfahren nicht vorgesehen und auch nicht geeignet.

Der vorliegenden Erfindung liegt die Aufgabe zugrunde, die vorgenannten Nachteile der bekannten Verfahren zu vermeiden und ein einfacheres Verfahren zur mikrobiologischen Bodenreinigung zu entwickeln, das geringen Energiebedarf für die Suspendierung zur homogenen Bodenbehandlung bei hohen Feststoffgehalten der Bodensuspenison aufweist, bei dem für den biologischen Schadstoffabbau nur ein Bioreaktor für das Feingut erforderlich ist und mit dem insbesondere auch polyzyklische aromatische Kohlenwasserstoffe aus schluffhaltigen Böden abzuscheiden sind.

Diese Aufgabe wird ausgehend von einem Verfahren zur mikrobiologischen Bodenreinigung der eingangs genannten Gattung durch die kennzeichnenden Merkmale von Patentanspruch 1 gelöst.

Weiterbildungen des Verfahrens sind in den Unteransprüchen 2 bis 5 niedergelegt.

Eine Vorrichtung zur Durchführung des Verfahrens weisen die Merkmale von Anspruch 6 aus.

In den Ansprüchen 7 und 8 sind Ausgestaltungen von Bioreaktoren gemäß Anspruch 6 ausgewiesen.

Bei einer Behandlung von kontaminierten Böden nach dem erfindungsgemäßen Verfahren und unter Anwendung der erfindungsgemäßen Vorrichtung lassen sich folgende Vorteile erreichen:
- geringer Energiebedarf für die Suspendierung zur homogenen Bodenbehandlung bei Feststoffkonzentrationen bis zu 60 % der Bodensuspension;
- optimale Auflösung der Bodenanteile in der Suspensionsflüssigkeit, so daß auch die Bodenanteile gut voneinander getrennt in der Bodensuspension vorliegen;
- durch die Behandlung des optimal aufgeschlossenen und aufgetrennten Bodens in der Suspension im Bioreaktor wird die Bioverfügbarkeit der im Boden vorhandenen Schadstoffe wesentlich verbessert;
- die Stoffaustauschvorgänge in dem suspendierten Bodenmaterial werden sehr stark beschleunigt und
- auch vergleichsweise schwer lösliche Schadstoffe sind den Mikroorganismen auch in Böden mit hohen Schluffanteilen direkt und unmittelbar zugänglich.

Der gereinigte Boden kann nach der Entwässerung, gegebenenfalls einschließlich der zuvor abgetrennten, groben Bestandteile, wiederverwendet werden. Die Prozessflüssigkeit mit den darin enthaltenden Mikroorganismen kann weitgehend in umweltfreundlicher Weise innerhalb des Prozesses im Kreislauf geführt werden. Das Verfahren und die Vorrichtung sind auch zur Behandlung solcher Böden geeignet, die an Kokereistandorten mit schwer abbaubaren Kohlenwasserstoffen belastet sind. Beispielsweise gelingt es, die PAK-Verunreinigungen (PAK = polyzyklische aromatische Kohlenwasserstoffe) von Kokereiböden mit hohem Schluffanteil innerhalb nur weniger Wochen weitgehend abzubauen. Solche Böden mit sehr hohem Feinstkornanteil ließen sich bisher nur unvollständig durch die bekannten biologischen Dekontaminierungsmaßnahmen behandeln und sanieren.

Die Erfindung wird nachfolgend anhand der Zeichnung und eines Beispiels näher beschrieben.

Es zeigen
- Figur 1: das erfindungsgemäße Verfahren zur mikrobiologischen Bodenreinigung in einer schematischen Übersichtsdarstellung,
- Figur 2: eine Bioreaktoranlage mit zugeordnetem Begasungsbehälter und Meß- und Regelzubehör, ebenfalls in einer schematischen Darstellung,
- Figur 3: eine andere Ausgestaltung der Bioreaktoranlage,
- Figur 4: ein Gaschromatogramm eines unbehandelten Kokereibodens und
- Figur 5: ein Gaschromatogramm des erfindungsgemäß behandelten Kokereibodens.

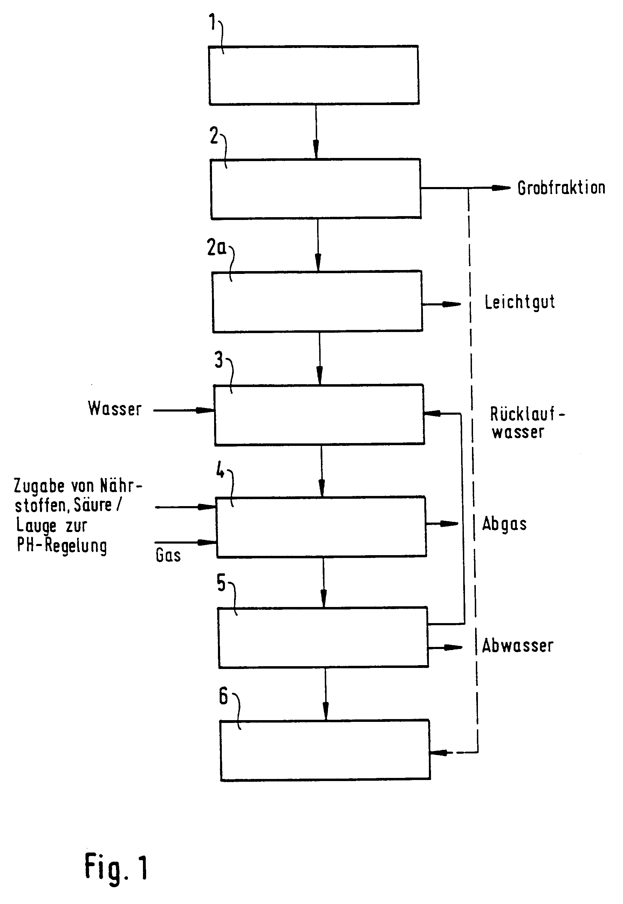

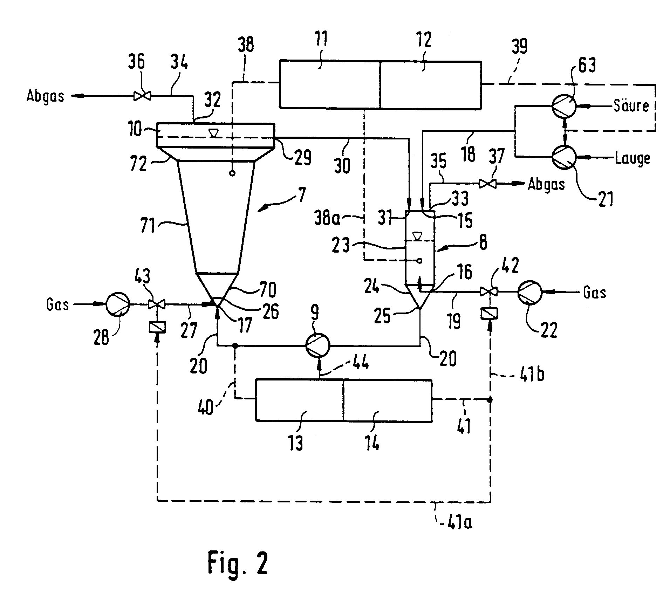

In Figur 1 ist ein Verfahrensschema zur mikrobiologischen Bodenreinigung gemäß der Erfindung abgebildet. Der Bodenaushub 1 wird einer Siebanlage 2 zugeführt. Die dort abgesiebte Grobfraktion wird gegebenenfalls anderweitig entsorgt. Wenn die Grobfraktion nicht kontaminiert ist, kann sie auch dem Feingut nach dessen abgeschlossener Dekontaminierung wieder zugemischt werden. Das Feingut aus der Siebanlage 2 wird bedarfsweise einer Sortieranlage 2a aufgegeben und darin von Leichtgut abgetrennt. Anschließend gelangt das Feingut in eine Läutertrommel 3 und wird dort in Wasser suspendiert. In die Läutertrommel 3 kann ein Teil der Suspensionsflüssigkeit aus dem Prozess im Kreislauf zurückgeführt werden. Die Bodensuspension aus der Läutertrommel 3 gelangt in eine Bioreaktoranlage 4, die aus einem Sprudelbettreaktor 7 und einem zugehörigen Begasungsbehälter 8 besteht (Figur 2). In die Bioreaktoranlage 4 wird Bodensuspension aus der Läutertrommel 3 mit Nährstoffe für die Mikroorganismen, Säure bzw. Lauge zur pH-Wert-Regelung und mit Gas beaufschlagt, um möglichst optimale Lebensbedingungen für die Mikroorganismen zu schaffen, die die verschiedenen Schadstoffe dadurch abbauen, daß sie diese biologisch in unschädliche Bestandteile auftrennen bzw. umwandeln. Überraschenderweise kann die Feststoffkonzentration beim erfindungsgemäßen Verfahren bis zu 60 % betragen.

Gasförmige Umwandlungsprodukte werden mit der Abluft ausgetragen und im Bedarfsfalle einem gesonderten Reinigungsprozess in einer in der Zeichnung nicht dargestellten Abluft-Filteranlage unterworfen. Die Bodensuspension wird in der Bioreaktoranlage 4 durch Umpumpen von dekantierter Suspensionsflüssigkeit in Suspension gehalten. Die Zeitdauer der Behandlung richtet sich nach der Art der Kontamination des Bodens in folgender Staffelung:

| | |
|---|---|
| mineralölbelastete Böden | 2 bis 5 Tage, |
| leichtabbaubare PAK | 5 bis 10 Tage und |
| schwerabbaubare PAK | 10 bis 20 Tage und mehr. |

Zu Beginn dieser Zeitspannen wird jeweils überprüft, ob der vorgegebene Dekontaminierungsgrad erreicht ist.

Nach Erreichen des vorgegebenen Dekontaminierungsgrades wird die Bodensuspension im Sprudelbettreaktor 7 der Bioreaktoranlage 4 eingedickt und dann einer Entwässerungsanlage 5 zugeführt, dessen Abwasser teilweise als Suspensionsflüssigkeit innerhalb des Prozesses wiederverwendet wird. Aus dem Prozess auszuschleusendes Abwasser ist einer anderweitigen Entsorgung zuzuführen. Das entwässerte Feingut wird, gegebenenfalls zusammen mit der zuvor abgetrennten Grobfraktion, wiederverwendet und zur Bodenablagerung 6 gebracht.

In Figur 2 ist die Bioreaktoranlage 4, bestehend aus dem hydraulisch angeströmten Sprudelbettreaktor 7 mit dem zugeordneten Begasungsbehälter 8, in einer Übersichtsdarstellung abgebildet. Der Sprudelbettreaktor 7 besteht aus einem konischen Behälterteil 70, in den die Suspensionsflüssigkeit von unten über eine Flüssigkeitsleitung 20 aufgegeben wird. Behälterteil 70 geht in einen weiteren konischen Behälterteil 71 über, in dem der Bioabbau in den einzelnen sich dort ausbildenden Abbauzonen gleichfälliger Kornbereiche, die sich teilweise überschneiden, erfolgt. Die Behälterteile 70 und 71 dienen, nachdem der biologische Schadstoffabbau beendet worden ist, als Eindickbehälter für die Bodensuspension. Daran schließt ein weiterer konischer Behälterteil 72 an, in dessen unteren Bereich sich eine Sedimentationszone aufbaut. Der obere Teil des Sprudelbettreaktors 7 wird von einen Rundbehälter 10 gebildet. Im Begasungsbehälter 8 ist ein Säure/Lauge-Einlaß 15 vorgesehen, an den eine Säure/Lauge-Leitung 18 angeschlossen ist. Die Säure bzw. Lauge wird von einer Säurepumpe 63 bzw. Laugepumpe 21 in den Begasungsbehälter 8 gefördert. Im Begasungsbehälter 8 ist weiterhin ein Gaseinlauf 16 angebracht, an den eine Gasleitung 19 anschließt. Das Gas wird über ein Gebläse 22 in den Begasungsbehälter 8 eingetragen. Es löst sich in der Suspensionsflüssigkeit. Schließlich verfügt der Begasungsbehälter 8 über einen Flüssigkeitsauslaß 25, an den die Flüssigkeitsleitung 20 anschließt, über die mittels einer Flüssigkeitspumpe 9 die begaste Suspensionsflüssigkeit aus dem Begasungsbehälter 8 in den Sprudelbettreaktor 7 gefördert wird, in den sie über einen Flüssigkeitseinlaß 17 am Behälterteil 70 hydraulisch eingetragen wird. Der Flüssigkeitsauslaß 25 ist an einem Spitzboden 24 angeordnet, der den Begasungsbehälter 8, der im übrigen als Rundbehälter 23 ausgebildet ist, nach unten abschließt. In den konischen Behälterteil 70 des Sprudelbettreaktors 7 ist ein Gaseinlaß 26 seitlich eingelassen, an den eine Gasleitung 27 anschließt, die mit einem Gebläse 28 versehen ist, das bei Bedarf zusätzliches Gas in den Sprudelbettreaktor 7 hineindrückt, das sich in der Suspensionsflüssigkeit löst. Der Rundbehälter 10 des Sprudelbettreaktors 7 weist einen Flüssigkeitsauslaß 29 auf, an den eine Flüssigkeitsleitung 30 anschließt, aus der die dekantierte Flüssigkeit aus dem Sprudelbettreaktor 7 über einen Flüssigkeitseinlaß 31 in den Begasungsbehälter 8 gelangt. Der Sprudelbettreaktor 7 und der Begasungsbehälter 8 verfügen weiterhin über je einen Abgasauslaß 32 bzw. 33 für je eine Abgasleitung 34 bzw. 35, die mit Ventilen 36 bzw. 37 bestückt sind. Das Abgas wird, wie bereits erwähnt, einer anderweitigen Entsorgung zugeführt. Der Begasungsbehälter 8 ist über eine Meßleitung 38a und der Sprudelbettreaktor 7 mit einer Meßleitung 38 mit einer Meßeinrichtung 11 verbunden. Der Meßeinrichtung 11 ist eine Regeleinrichtung 12 zugeordnet, die über eine Regelleitung 39 mit der Säurepumpe 63 bzw. der Laugepumpe 21 verbunden ist. Über die Meßleitung 38 werden die Temperatur und der O₂-Gehalt der Bodensuspension im Sprudelbettreaktor 7 und über die Meßleitung 38a der pH-Wert der Suspensionsflüssigkeit im Begasungsbehälter 8 von hier nicht dargestellten Meßwertgebern ermittelt und der Meßeinrichtung 11 übermittelt. Über die Regeleinrichtung 12 wird der pH-Wert konstant gehalten oder geänderten Bedingungen angepaßt, indem die Säure/Lauge-Zufuhr in den Begasungsbehälter 8 über die Regelleitung 39, die zur Säurepumpe 63 bzw. zur Laugepumpe 21 führt, eingeregelt wird. Weiterhin sind der Sprudelbettreaktor 7 sowie der Begasungsbehälter 8 meß- und regelungstechnisch an eine weiteren Meßeinrichtung 13 und zugehörige Re-geleinrichtung 14 angeschlossen. Über eine Meßleitung 40, die von der Flüssigkeitsleitung 20 zur Meßeinrichtung 13 führt, werden Meßwerte von Strömung, Druck und O₂-Gehalt der Suspensionsflüssigkeit in der Flüssigkeitsleitung 20 über geeignete Meßwertgeber, die hier nicht dargestellt sind, ermittelt und der Meßeinrichtung 13 übermittelt. Von der angeschlossenen Regeleinrichtung 14 führt eine Regelleitung 41, 41a zum Ventil 43 in der Gasleitung 27 des Sprudelbettreaktors 7 sowie eine Regelleitung 41b zum Ventil 42 der Gasleitung 19 des Begasungsbehälters 8 zwecks Einregelung des O₂-Gehaltes. Außerdem führt eine Regelleitung 44 zur Flüssigkeitspumpe 9 in der Flüssigkeitsleitung 20, um die Flüssigkeitszufuhr nach Druck und Menge dem jeweiligen Bedarf des Sprudelbettreaktors 7 entsprechend zu regulieren. Der Sprudelbettreaktor 7 gemäß Fig. 2 ist wegen seiner konischen Ausbildung insbesondere für Feinfraktionen mit einem breiten Kornspektrum von 0 bis 0,5 mm Korndurchmesser geeignet.

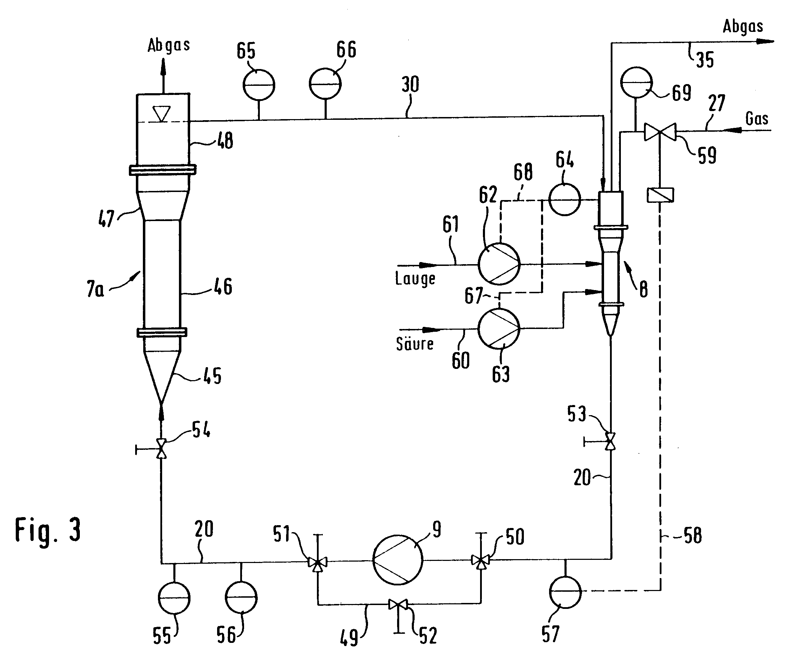

In Figur 3 ist ein abgewandelter Sprudelbettreaktor 7a dargestellt, bei dem an einen kurzen konischen Behälterteil 45 ein längerer zylindrischer Behälterteil 46 anschließt, an dem ein zweiter konischer Behälterteil 47 befestigt ist, der mit einem zweiten zylindrischen Behälterteil 48 verbunden ist. Dieser abgewandelte Sprudelbettreaktor 7a ist insbesondere für Feinfraktionen mit einem engen Kornspektrum von 20 »m bis 300 »m Korndurchmesser geeignet. Der Spudelbettreaktor 7a in der Ausgestaltung gemäß Fig. 3 ist über Flüssigkeitsleitung 20 sowie über Flüssigkeitsleitung 30 mit dem Begasungsbehälter 8 verbunden. Flüssigkeitsleitung 20 fördert die begaste Suspensionsflüssigkeit aus dem Begasungsbehälter 8 zum konischen Behälterteil 45 des Sprudelbettreaktors 7a. Flüssigkeitsleitung 30 führt die Überschußflüssigkeit aus dem zylindrischen Behälterteil 48 des Sprudelbettreaktors 4 zurück in den Begasungsbehälter 8. In die Flüssigkeitsleitung 30 sind ein Temperaturmeßgerät 65 und ein Sauerstoffmeßgerät 66 integriert. Die Flüssigkeitsleitung 20 enthält ein unterhalb des Begasungsbehälters 8 angeordnetes Absperrventil 53 sowie ein unterhalb des Sprudelbettreaktors 7a angeordnetes Absperrventil 54. Weiterhin ist in die Flüssigkeitsleitung 20 eine Flüssigkeitspumpe 9 integriert, welche von einer ein Absperrventil 52 enthaltenden Umgehungsleitung 49 über der Flüssigkeitspumpe 9 vor- bzw. nachgeschaltete Dreiwegeventile 50, 51 umgehbar ist, wenn die Flüssigkeitspumpe 9 nicht oder zeitweilig nicht betrieben wird. Weiterhin sind in der Flüssigkeitsleitung 20 ein Temperaturmeßgerät 55, ein Durchflußmeßgerät 56 sowie ein Sauerstoffmeßgerät 57 untergebracht. Letzteres ist über eine Regelleitung 58 mit einem Absperrventil 59 in der Gasleitung 27 verbunden und bildet mit dem Ventil 59 einen Sauerstoff-Regelkreis. Der Gasleitung 27 ist weiterhin ein Durchflußmeßgerät 69 zugeordnet. Aus dem Begasungsbehälter 8 führt eine Abgasleitung 35 heraus, die zu einer in der Zeichnung nicht dargestellten Filteranlage führt. An dem Begasungsbehälterteil 8 sind eine Säureleitung 60 mit Säurepumpe 63 sowie eine Laugeleitung 61 mit Laugepumpe 62 sowie ein pH-Wert-Meßgerät 64 angeschlossen. Letzteres ist über Regelleitungen 67, 68 mit den Pumpen 62, 63 verbunden und bildet mit diesen pH-Wert-Regelkreise.

Der erfindungsgemäße Betrieb des Sprudelbettreaktors 7, 7a sieht eine an den Bedarf des biologischen Stoffabbaus angepaßte Sauerstoffversorgung vor. Für den aeroben Abbau ist bei hohem O₂-Bedarf ein O₂-Eintrag durch Begasung mit Luft oder Reinsauerstoff im Begasungsbehälter 8 und im Sprudelbettreaktor 7, 7a vorgesehen. Bei geringem O₂-Bedarf ist eine Begasung allein im Begasungsbehälter 8 ausreichend. Bei sehr geringem O₂-Bedarf kann zeitweise auf die Umwälzung der Flüssigkeit verzichtet werden. Weil dies ein langsames Sedimentieren der Bodensuspension im Sprudelbettreaktor 7, 7a und eine Abnahme des O₂-Gehaltes in der Bodensuspension zur Folge hat, muß die Flüssigkeitsumwälzung von Zeit zu Zeit aktiviert werden.

Im Sprudelbettreaktor 7, 7a können wechselweise aerobe und anaerobe Bedingungen in der Bodensuspension eingestellt werden, so daß auch solche Bedingungen geschaffen werden können, die für den biologischen Abbau von höher halogenierten Kohlenwasserstoffen erforderlich sind.

### Beispiel 1

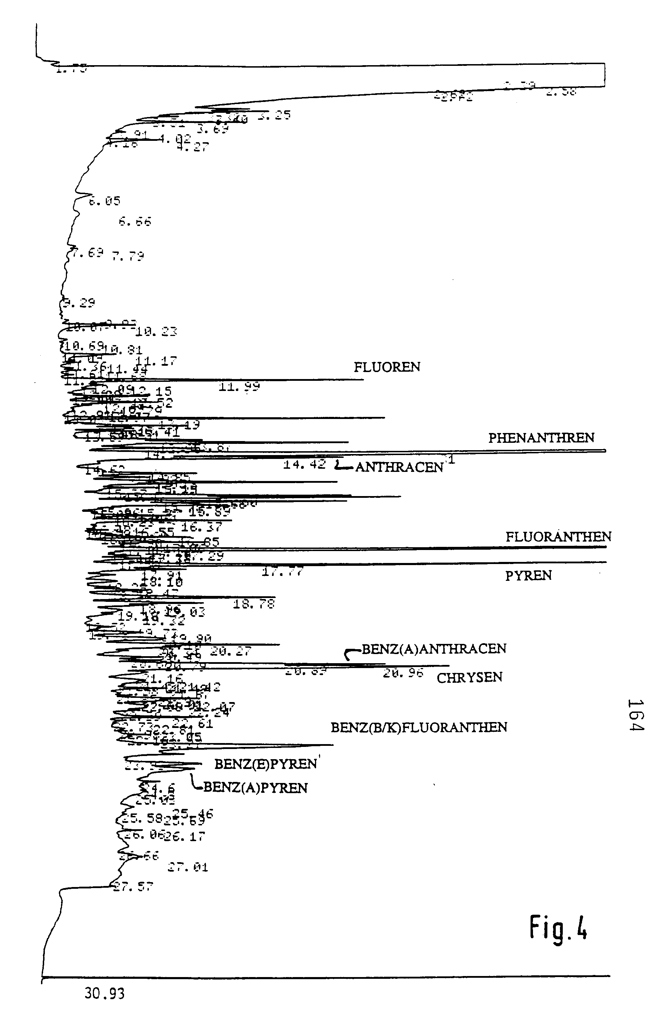

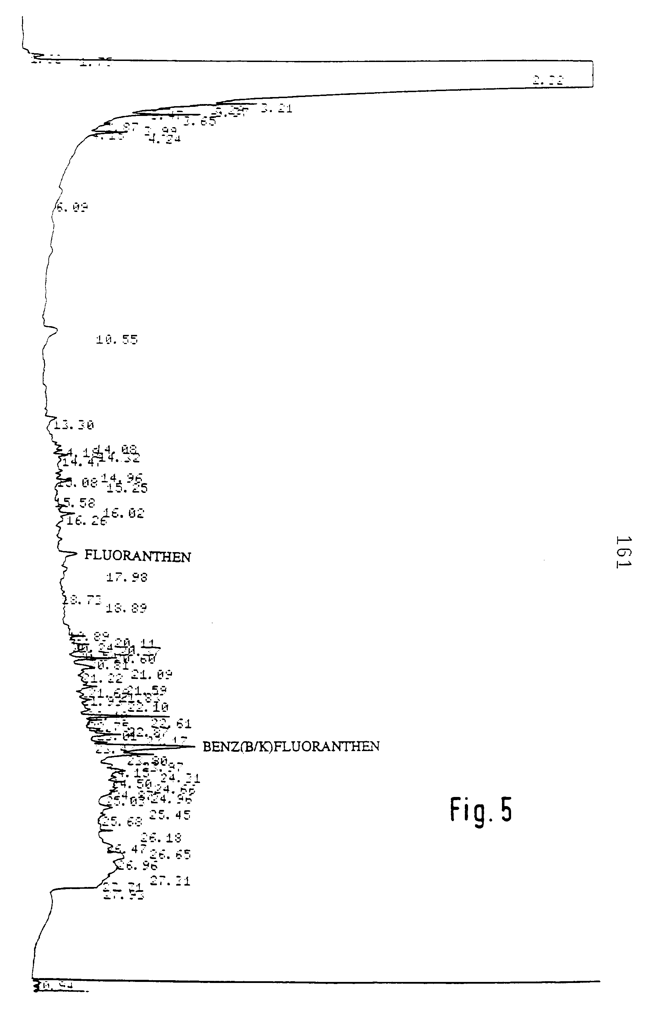

Für die Behandlung eines Bodens mit einem Korndurchmesser < 300 »m und einer Partikeldichte von 2,6 g/cm³ ist eine Anströmgeschwindigkeit von 4x10 ⁻⁴ bis 8x10 ⁻⁴ m/s als optimal ermittelt worden. Die Feststoffkonzentration im Sprudelbettreaktor 7, 7a betrug dabei 500 g/l, während die Feststoffkonzentration der im Kreislauf geführten Suspensionsflüssigkeit < 50 g/l betrug. Das Bodenmaterial wurde von einem Standort einer ehemaligen Kokerei entnommen. Die durchgeführten biologischen Abbauversuche hatten zum Ergebnis, daß die im Boden ursprünglich enthaltenen PAK (PAK = polyzyklische aromatische Kohlenwasserstoffe), deren Anteile und Verteilung aus dem Gaschromatogramm gemäß Figur 4 entnommen werden können, nach einer Behandlung von 7 Tagen fast vollständig abgebaut werden konnten, wie das Gaschromatogramm der Figur 5 zeigt. Die Figuren 4 und 5 weisen aus, auch wenn man nicht im Einzelnen jedem auftretendem PAK seinen charakteristischen Schadstoffanteil zuordnet, daß insgesamt eine fast vollständige Dekontamination erreicht werden konnte.

Die Konzentration der üblicherweise gemäß der EPA-"List of priority pollants" (M.A. Callahan et al.(1979), Water-related environmental fate of 129 priority pollutants, EPA-Report -440/4-79-0296) gemessenen PAK wurde im Beispiel von 180 mg PAK/kg Boden auf 18 mg PAK/kg Boden reduziert.

### BEZUGSZEICHENLISTE

- 1: Bodenaushub
- 2: Siebanlage
- 2a: Sortieranlage
- 3: Läutertrommel
- 4: Bioreaktoranlage
- 5: Entwässerungsanlage
- 6: Bodenablagerung
- 7: Sprudelbettreaktor
- 7a: Sprudelbettreaktor
- 8: Begasungsbehälter
- 9: Flüssigkeitspumpe
- 10: Rundbehälter
- 11: Meßeinrichtung
- 12: Regeleinrichtung
- 13: Meßeinrichtung
- 14: Regeleinrichtung
- 15: Säure/Lauge-Einlaß
- 16: Gaseinlaß
- 17: Flüssigkeitseinlaß
- 18: Säure/Lauge-Leitung
- 19: Gasleitung
- 20: Flüssigkeitsleitung
- 21: Laugepumpe
- 22: Gebläse
- 23: Rundbehälter
- 24: Spitzboden
- 25: Flüssigkeitsauslaß
- 26: Gaseinlaß
- 27: Gasleitung
- 28: Gebläse
- 29: Flüssigkeitsauslaß
- 30: Flüssigkeitsleitung
- 31: Flüssigkeitseinlaß
- 32: Abgasauslaß
- 33: Abgasauslaß
- 34: Abgasleitung
- 35: Abgasleitung
- 36: Ventil
- 37: Ventil
- 38: Meßleitung
- 38a: Meßleitung
- 39: Regelleitung
- 40: Meßleitung
- 41: Regelleitung
- 41a: Regelleitung
- 41b: Regelleitung
- 42: Ventil
- 43: Ventil
- 44: Regelleitung
- 45: konischer Behälterteil
- 46: zylindrischer Behälterteil
- 47: konischer Behälterteil
- 48: zylindrischer Behälterteil
- 49: Umgehungsleitung
- 50: Dreiwegeventil
- 51: Dreiwegeventil
- 52: Absperrventil
- 53: Absperrventil
- 54: Absperrventil
- 55: Temperaturmeßgerät
- 56: Durchflußmeßgerät
- 57: Sauerstoffmeßgerät
- 58: Regelleitung
- 59: Absperrventil
- 60: Säureleitung
- 61: Laugeleitung
- 62: Laugepumpe
- 63: Säurepumpe
- 64: pH-Wert-Meßgerät
- 65: Temperaturmeßgerät
- 66: Sauerstoffmeßgerät
- 67: Regelleitung
- 68: Regelleitung
- 69: Durchflußmeßgerät
- 70: konischer Behälterteil
- 71: konischer Behälterteil
- 72: konischer Behälterteil

## Patentansprüche

1. Verfahren zur mikrobiologischen Bodenreinigung, wobei der verunreinigte Boden ausgehoben und in eine Grobfraktion und eine Feinfraktion getrennt wird, die Feinfraktion in einem Bioreaktor dekontaminiert wird, Betriebswasser in den Kreislauf zurückgeführt wird und der dekontaminierte Boden, gegebenenfalls nach Wiederzumischung der Grobfraktion, wieder angefüllt wird, **dadurch gekennzeichnet**, daß
a) eine Abtrennung des Grobmaterials > 0,5 mm in einer Siebstufe erfolgt, gegebenenfalls Leichtgut in einer Sortierstufe abgetrennt,
b) die Feinfraktion < 0,5 mm in einer Läuterstufe suspendiert wird,
c) der biologische Schadstoffabbau der Bodensuspension mittels aerober oder anaerober Mikroorganismen in einer Bioreaktoranlage, bestehend aus einem hydraulisch betriebenen Sprudelbettreaktor und aus einem diesem zugeordneten Begasungsbehälter, erfolgt, deren Betriebsbedingungen durch Regelkreise für Temperatur, pH-Wert, O₂-Gehalt der Bodensuspension sowie Druck und Geschwindigkeit der Suspensionsflüssigkeit überwacht werden,
d) die Bodensuspension in der Bioreaktoranlage durch Umpumpen von dekantierter Suspensionsflüssigkeit in Suspension gehalten wird, bis der vorgegebenen Dekontaminationsgrad erreicht ist,
e) die Bodensuspenion im Sprudelbettreaktor eingedickt und
f) in einer Entwässerungsstufe entwässert wird.

2. Verfahren nach Anspruch 1, **dadurch gekennzeichnet,** daß die Behandlung in der Bioreaktoranlage mittels anaerober Mikroorganismen erfolgt und in einer anaerob eingestellten Bodensuspension dekontaminiert wird, wobei der Begasungsbehälter und/oder Sprudelbettreaktor mit einem inerten Gas beschickt wird.

3. Verfahren nach Anspruch 1 und 2, **dadurch gekennzeichnet,** daß die Bodensuspension wechselweise anaerob und aerob eingestellt wird.

4. Verfahren nach Anspruch 1 oder 2, **dadurch gekennzeichnet**, daß Flüssigkeit aus der Entwässerungsstufe in die Läuterstufe zurückgeführt wird.

5. Verfahren nach einem der Ansprüche 1 bis 3, **dadurch gekennzeichnet**, daß das Abgas aus dem Sprudelbettreaktor und aus dem Begasungsbehälter in einer nachgeschalteten Filterstufe gereinigt wird.

6. Vorrichtung zur Durchführung der Verfahren nach den Ansprüchen 1 bis 5, **dadurch gekennzeichnet**, daß eine Siebanlage (2) zur Abtrennung der Grobfraktion, eine Sortieranlage (2a) zur Abscheidung des Leichtgutes und eine Läutertrommel (3) zur Suspendierung der Feinfraktion angeschlossen sind, die mit einer Bioreaktoranlage (4) zum biologischen Schadstoffabbau, bestehend aus einem Sprudelbettreaktor (7, 7a) mit zugeordnetem Begasungsbehälter (8) sowie Meß- und Regeleinrichtungen (11, 12, 13, 14) vorgeschaltet sind sowie eine Entwässerungsanlage (5) nachgeschaltet ist.

7. Vorrichtung nach Anspruch 6, **dadurch gekennzeichnet**, daß der Sprudelbettreaktor aus drei übereinander angeordneten konischen Behälterteilen (70, 71, 72) besteht, die in einen Rundbehälter (10) übergehen, im unteren Behälterteil (70) ein Gaseinlaß (26) für eine Gasleitung (27) mit Gebläse (28) und ein Flüssigkeitseinlaß (17) für eine Flüssigkeitsleitung (20) mit Flüssigkeitspumpe (9) angeordnet sind, der Sprudelbettreaktor (7) über die Flüssigkeitsleitung (20) mit dem Begasungsbehälter (8) verbunden ist, der als Rundbehälter (23) mit Spitzboden (24) und Flüssigkeitsauslaß (25) sowie mit Gaseinlaß (16) für Gasleitung (19) mit Gebläse (22) ausgebildet ist, der Rundbehälter (10) des Sprudelbettreaktors (7) mit einem Flüssigkeitsauslaß (29) für eine Flüssigkeitsleitung (30) versehen ist, die die Suspensionsflüssigkeit über einen Flüssigkeitseinlaß (31) in den Begasungsbehälter (8) einträgt, der Begasungsbehälter (8) weiterhin über einen Säure/Laugeeinlaß (15) verfügt, an die eine Säure/Laugeleitung (18) anschließt, die zu einer Säurepumpe (63) und zu einer Laugepumpe (21) führt, der Sprudelbettreaktor (7) und der Begasungsbehälter (8) je einen Abgasauslaß (32, 33) für je eine Abgasleitung (34, 35) mit Absperrventil (36, 37) aufweisen, der Sprudelbettreaktor (7) und der Begasungsbehälter (8) meß- und regeltechnisch an eine Meßeinrichtung (11) und eine Regeleinrichtung (12) angeschlossen sind, wobei der Sprudelbettreaktor (7) über eine Meßleitung (38) und der Begasungsbehälter (8) über eine Meßleitung (38a) mit der Meßeinrichtung (11) und der Regeleinrichtung (12) und letztere über eine Regelleitung (39) mit der Säurepumpe (63) und der Laugepumpe (21) verbunden sind und der Sprudelbettreaktor (7) sowie der Begasungsbehälter (8) außerdem meß- und regeltechnisch an eine weitere Meßeinrichtung (13) über eine Meßleitung (40), die zur Flüssigkeitsleitung (20) führt, sowie an eine weitere Regeleinrichtung (14), die über eine Regelleitung (41) zu Ventilen (43, 42) der Gasleitungen (19, 27) und über eine Regelleitung (44) zur Flüssigkeitspumpe (9) in der Flüssigkeitsleitung (20) führt, angeschlossen sind.

8. Vorrichtung nach Anspruch 6, **dadurch gekennzeichnet**, daß der Sprudelbettreaktor aus einem konischen Behälterteil (45), einem daran anschließenden zylindrischen Behälterteil (46), einem weiteren konischen Behälterteil (47) und einem daran anschließenden weiteren zylindrischen Behälterteil (48) besteht und dem Sprudelbettreaktor (7a) der Begasungsbehälter (8) zugeordnet ist, die über eine Absperrventile (53, 54) enthaltende Flüssigkeitsleitung (20) verbunden sind, in die eine Flüssigkeitspumpe (9) integriert ist, welche von einer ein Absperrventil (52) enthaltenden Umgehungsleitung (49) über Dreiwegeventile (50, 51) umgehbar ist, der Flüssigkeitsleitung (20) ein Temperaturmeßgerät (55), ein Durchflußmeßgerät (56) und ein Sauerstoffmeßgerät (57) zugeordnet sind, letzteres über eine Regelleitung (58) mit einem Absperrventil (59) in der Gasleitung (27) einen Sauerstoff-Regelkreis bildet, an dem Begasungsbehälter (8) eine Säureleitung (60) mit Säurepumpe (63) und eine Laugeleitung (61) mit Laugepumpe (62) sowie ein pH-Wert-Meßgerät (64) angeschlossen sind und letzteres über Regelleitungen (67, 68) mit den Pumpen (62, 63) pH-Wert-Regelkreise bildet sowie der Sprudelbettreaktor (7a) und der Begasungsbehälter (8) über eine Flüssigkeitsleitung (30) mit zugeordnetem Temperaturmeßgerät (65) und Sauerstoffmeßgerät (66) verbunden ist.

## Claims

1. A process for microbiological soil decontamination, in which the contaminated soil is dug out and is separated into a coarse fraction and a fine fraction, and the fine fraction is decontaminated in a bioreactor, water from the process is returned to the circuit and the decontaminated soil is filled in once more, optionally after the coarse fraction has been mixed back in, **characterised in that**
a) the coarse material > 0.5 mm is separated off in a sieving stage and optionally light material is separated off in a sorting stage;
b) the fine fraction < 0.5 mm is suspended in a washing stage;
c) biological decomposition of contaminants in the soil suspension takes place using aerobic or anaerobic micro-organisms in a bioreactor installation comprising a hydraulically operated spouted-bed reactor and a gassing container associated therewith, and the operational conditions thereof are monitored by control circuits for temperature, pH value and O₂ content of the soil suspension, and pressure and speed of the suspension liquid;
d) the soil suspension is kept in suspension in the bioreactor installation by re-circulating decanted suspension liquid, until the pre-determined degree of decontamination is achieved;
e) the soil suspension is concentrated in the spouted-bed reactor; and
f) drying takes place in a drying stage.

2. A process according to claim 1, **characterised in that** treatment in the bioreactor installation takes place using anaerobic micro-organisms and decontamination takes place in an anaerobically adjusted soil suspension, the gassing container and/or spouted-bed reactor being supplied with an inert gas.

3. A process according to claims 1 and 2, **characterised in that** the soil suspension is adjusted alternately anaerobically and aerobically.

4. A process according to claim 1 or 2, **characterised in that** liquid from the drying stage is returned to the washing stage.

5. A process according to one of claims 1 to 3, **characterised in that** the waste gas from the spouted-bed reactor and the gassing container is purified in a filtering stage arranged downstream thereof.

6. A device for carrying out the process according to claims 1 to 5, **characterised in that** a sieving installation (2) for separating off the coarse fraction, a sorting installation (2a) for removing light material and a washing drum (3) for suspending the fine fraction are connected, and are arranged in series together with a bioreactor installation (4) which is for biological decomposition of contaminants and which comprises a spouted-bed reactor (7, 7a) with an associated gassing container (8) and measuring and control devices (11, 12, 13, 14), and a drying installation (5) is arranged downstream thereof.

7. A device according to claim 6, **characterised in that** the spouted-bed reactor comprises three conical container parts (70, 71, 72) arranged one above another and passing into a round container (10);
- a gas inlet (26) for a gas line (27) with a fan (28), and a liquid inlet (17) for a liquid line (20) with a liquid pump (9), are arranged in the lower container part (70), and the spouted-bed reactor (7) is connected to the gassing container (8) via the liquid line (20), the gassing container being formed as a round container (23) with a pointed base (24) and a liquid outlet (25) and with a gas inlet (16) for a gas line (19) with a fan (22);
- the round container (10) in the spouted-bed reactor (7) is provided with a liquid outlet (29) for a liquid line (30) which introduces the suspension liquid into the gassing container (8) through a liquid inlet (31);
- the gassing container (8) also has an acid/lye inlet (15) to which there is connected an acid/lye line (18) which leads to an acid pump (63) and a lye pump (21);
- the spouted-bed reactor (7) and the gassing container (8) each have a waste gas outlet (32, 33) for a respective waste gas line (34, 35) with a shut-off valve (36, 37);
- the spouted-bed reactor (7) and the gassing container (8) are each connected for measurement and control to a measuring device (11) and a control device (12), the spouted-bed reactor (7) being connected via a measuring line (38) and the gassing container (8) being connected via a measuring line (38a) to the measuring device (11) and the control device (12), the latter devices being connected via a control line (39) to the acid pump (63) and the lye pump (21);
- the spouted-bed reactor (7) and the gassing container (8) are also connected for measurement and control firstly to a further measuring device (13) via a measuring line (40) leading to the liquid line (20), and secondly to a further control device (14) which itself leads via a control line (41) to valves (43, 42) in the gas lines (19, 27) and via a control line (44) to the liquid pump (9) in the liquid line (20).

8. A device according to claim 6, **characterised in that** the spouted-bed reactor comprises a conical container part (45), a cylindrical container part (46) connected thereto, a further conical container part (47) and a further cylindrical container part (48) connected thereto;
- the gassing container (8) is associated with the spouted-bed reactor (7) and is connected thereto via a liquid line (20) containing shut-off valves (53, 54), in which line a liquid pump (9) is integrated, and with the aid of three-way valves (50, 51) the said line can be by-passed via a by-pass line (49) containing a shut-off valve (52);
- a temperature-measuring device (55), a flow-measuring device (56) and an oxygen-measuring device (57) are associated with the liquid line (20), the oxygen-measuring device forming an oxygen-control circuit via a control line (58) with a shut-off valve (59) in the gas line (27);
- an acid line (60) with an acid pump (63), a lye line (61) with a lye pump (62), and a pH-value measuring device (64) are connected to the gassing container (8), and the said pH-value measuring device forms pH-value control circuits with the pumps (62, 63) via control lines (67, 68);
- the spouted-bed reactor (7a) and the gassing container (8) are connected via a liquid line (30) with an associated temperature-measuring device (65) and oxygen-measuring device (66).

## Revendications

1. Procédé de nettoyage microbiologique d'un sol par lequel le sol pollué est enlevé et séparé en une fraction grossière et en une fraction fine, la fraction fine est décontaminée dans un bioréacteur, l'eau industrielle est retournée dans la circulation et le sol décontaminé, le cas échéant après un remélange avec la fraction grossière, est à nouveau remblayé, caractérisé en ce que
(a) une séparation des matériaux grossiers > 0,5 mm a lieu dans une étape de criblage, le cas échéant la matière légère est séparée dans une étape de triage,
(b) la fraction fine < 0,5 mm est suspendue dans une étape de purification,
(c) la décomposition biologique des éléments polluants de la suspension de sol a lieu à l'aide de micro-organismes aérobies ou anaérobies, dans une installation de bioréacteur consistant en un réacteur à lit bouillonnant à conduite hydraulique et en un récipient d'introduction de gaz conjugué, dont les conditions de fonctionnement seront contrôlées à travers des circuits de réglage pour la température, la valeur du pH, la teneur en O₂ de la suspension de sol de même que la pression et la vitesse de l'écoulement de la suspension,
(d) la suspension de sol, dans l'installation de bioréacteur, est maintenue en suspension au moyen d'un transvasement du liquide de la suspension décantée, jusqu'à ce que le degré de décontamination prédéterminé soit atteint,
(e) la suspension de sol est concentrée dans le réacteur à lit bouillonnant et
(f) est asséchée dans une étape de déshydratation.

2. Procédé selon la revendication 1, caractérisé en ce que le traitement dans l'installation de bioréacteur a lieu à l'aide de micro-organismes anaérobies et est décontaminé dans une suspension de sol ajustée en anaérobies, le récipient d'introduction de gaz et/ou le réacteur à lit bouillonnant étant alimenté avec un gaz inerte.

3. Procédé selon la revendication 1 et 2, caractérisé en ce que la suspension de sol est ajustée tour à tour en anaérobies et en aérobies.

4. Procédé selon la revendication 1 ou 2, caractérisé en ce que le liquide est retourné, depuis l'étape de déshydratation, dans l'étape de purification.

5. Procédé selon l'une des revendications 1 à 3, caractérisé en ce que le gaz sortant du réacteur à lit bouillonnant et du récipient d'introduction de gaz est purifié dans une étape de filtration placée en aval.

6. Dispositif pour mettre en oeuvre le procédé selon les revendications 1 à 5, caractérisé en ce qu'un dispositif de tamis (2) pour séparer la fraction grossière, un dispositif de triage (2a) pour extraire la matière légère et un tambour de purification (3) pour suspendre la fraction fine sont reliés en série et placés en amont d'un dispositif de bioréacteur (4) pour la décomposition biologique des éléments polluants, consistant en un réacteur à lit bouillonnant (7,7a) avec un récipient d'introduction de gaz conjugué (8) de même que des moyens de mesure et de régulation (11,12,13,14), et qu'une installation de déshydratation (5) est placée en aval du dispositif de bioréacteur.

7. Dispositif selon la revendication 6, caractérisé en ce que le réacteur à lit bouillonnant consiste en trois éléments de récipient coniques disposés les uns au-dessus des autres (70,71,72), qui débouchent dans un récipient rond (10), dans l'élément inférieur du récipient (70) sont disposés une entrée pour le gaz (26) pour une conduite de gaz (27) comportant un ventilateur (28) et une entrée pour le liquide (17) pour une conduite de liquide (20) comportant une pompe à liquide (9), le réacteur à lit bouillonnant (7) est relié par l'intermédiaire de la conduite de liquide (20) au récipient d'introduction de gaz (8), qui est construit comme un récipient rond (23) avec un corps pointu (24) et une sortie pour le liquide (25) de même qu'avec une entrée pour le gaz (16) pour une conduite de gaz (19) comportant un ventilateur (22), le récipient rond (10) du réacteur à lit bouillonnant (7) est muni d'une sortie pour le liquide (29), pour une conduite pour le liquide (30) qui transporte la suspension liquide sur une entrée du liquide (31) dans le récipient d'introduction de gaz (8), le récipient d'introduction de gaz (8) comporte également une entrée d'acide/de lessive alcaline (15), rattachée à une conduite d'acide/de lessive alcaline (18) qui conduit à une pompe à acide (63) et à une pompe à lessive alcaline (21), le réacteur à lit bouillonnant (7) et le récipient d'introduction de gaz (8) présentent chacun une sortie pour le gaz perdu (32,33) pour une conduite de gaz (34,35) respective comportant une soupape d'arrêt (36,37), le réacteur à lit bouillonnant (7) et le récipient d'introduction de gaz (8) sont reliés pour la mesure et la régulation à un dispositif de mesure (11) et à un dispositif de régulation (12), le réacteur à lit bouillonnant (7) étant relié par une conduite de mesure (38) et le récipient d'introduction de gaz (8) étant relié par une conduite de mesure (38a) au dispositif de mesure (11) et au dispositif de régulation (12) et ces derniers étant reliés par une conduite de régulation (39) à la pompe à acide (63) et à la pompe à lessive alcaline (21) et le réacteur à lit bouillonnant (7) ainsi que le récipient de dégazage (8), étant en outre raccordés pour la mesure et la régulation, à une installation de mesure supplémentaire (13) par une conduite de mesure (40) qui mène à la conduite de liquide (20), ainsi qu'à une installation de régulation supplémentaire (14) qui conduit par une conduite de régulation (41) à des soupapes (43,42) des conduites de gaz (19,27) et par une conduite de régulation (44) à une pompe à liquide (9) dans la conduite de liquide (20).

8. Dispositif selon la revendication 6, caractérisé en ce que le réacteur à lit bouillonnant consiste en un élément de récipient conique (45), un élément de récipient (46) cylindrique raccordé à ce dernier, un élément de récipient conique supplémentaire (47) et un élément de récipient cylindrique supplémentaire (48) rattaché à ce dernier et le récipient d'introduction de gaz est adjoint (8) au réacteur à lit bouillonnant (7a), qui sont reliés par une conduite de liquide contenant les soupapes d'arrêt (53,54), dans laquelle une pompe à liquide (9) est intégrée, laquelle est contournable au moyen de soupapes à trois voies (50,51) par une conduite de contournement (49) contenant une soupape d'arrêt (52), un appareil de mesure de la température (55), un appareil de mesure du débit (56) et un appareil de mesure de l'oxygène (57) sont associés dans la conduite de l'écoulement (20), l'appareil de mesure de l'oxygène formant, avec une conduite de régulation (58) comportant une soupape d'arrêt (59) dans la conduite de gaz (27) un circuit de régulation de l'oxygène, qu'au récipient d'introduction de gaz (8) sont reliés une conduite d'acide (60) avec une pompe à acide (63) et une conduite de lessive alcaline (61) comportant une pompe à lessive alcaline (62), de même qu'un appareil de mesure de la valeur du pH (64) et que ce dernier forme avec des conduites de régulation (67,68) avec les pompes (62,63) un circuit de régulation de la valeur du pH et que le réacteur à lit bouillonnant (7a) et le récipient d'introduction de gaz (8) sont reliés par une conduite de liquide (30) comportant un appareil de mesure de la température (65) et un appareil de mesure de l'oxygène (66) conjugués.
